# EUROPEAN PATENT APPLICATION

(11) **EP 4 736 758 A1**
(43) Date of publication of application: **06.05.2026**
(21) Application number: 25196745.1
(22) Date of filing: 19.08.2025
(51) Int. Cl.: A61B 5/145, A61B 5/1486

(54) **APPLICATOR FOR BIOMETRIC INFORMATION ACQUISITION**

(30) Priority: 31.10.2024 KR 20240152499
(71) Applicant: I-SENS, INC., Seoul 06646 (KR)
(72) Inventor: CHOI, Hyun Ho, 06646 Seoul (KR)
(74) Representative: BCKIP Part mbB

(57) **Abstract**

Provided is an applicator for biometric information acquisition, including a case forming an internal space and including an opening portion opened on one side, a plunger disposed in the internal space of the case and configured to move a sensor unit for biometric information acquisition toward the opening portion, a protection cap configured to shield the opening portion, and a sealing member disposed between the case and the protection cap to seal the internal space.

## Description

### CROSS-REFERENCE TO RELATED APPLICATION(S)

This application claims the benefit of Korean Patent Application No. 10-2024-0152499, filed on October 31, 2024, in the Korean Intellectual Property Office.

### BACKGROUND

### 1. Field of the Invention

Example embodiments relate to an applicator for biometric information acquisition.

### 2. Description of the Related Art

Various diseases occur to modern people, and a method for acquiring human biometric information is needed for proper treatment and management of these various diseases.

Diabetes is a representative example. To properly manage diabetes, a method to acquire blood glucose information, which is biometric information of diabetic patients, is needed.

Recently, a continuous glucose monitoring system (CGM) that is inserted in a human body to measure a value of the blood sugar every few minutes is being developed. In order to minimize a pain or an aversion of the user, which is caused by blood gathering, the CGM system may insert a needle-shaped sensor into a region such as a stomach and an arm at which the pain is relatively less and then continuously measure the blood sugar.

The CGM system includes a sensor unit that is attached to a body of a user to measure blood sugar information and transmits the measured blood sugar information, and a terminal that provides the blood sugar information received from the sensor unit to the user, allowing the user to monitor and manage the blood sugar information.

Here, the sensor unit is composed of a sensor module inserted in and attached to skin of the body of the user and measuring blood sugar from blood fluid of the user and a transmitter transmitting a value of the blood sugar measured by the sensor module to the terminal, and the sensor module is provided with a probe formed in a needle shape to be inserted into subcutaneous fat and to extract the interstitial fluid (ISF).

To insert and attach the sensor unit to the body, an applicator is used.

The applicator, as a configuration to insert and attach the sensor unit to the body, inserts and attaches the sensor unit to the body by the operation of the user, and by decoupling and removing the applicator from the body in a state in which the sensor unit is inserted and attached to the body, the sensor unit is allowed to remain attached to the body.

Since a portion of the sensor unit is inserted into the human body, the sensor unit installed inside the applicator and the internal space of the applicator may be kept sterile from the manufacturing stage until use by a consumer and maintained in a dehumidified state to protect electronic components. At the same time, the internal space of the applicator may be kept sealed from the outside to maintain a sterile and dehumidified state.

To maintain such a sterile, dehumidified, and sealed state, various packaging technologies are applied to the applicator, and minimizing packaging and reducing size are becoming one of objectives for applicator manufacturers.

In addition, to insert and attach the sensor unit to the body, the applicator propels the sensor unit in a direction toward the body of the user. During the process, constant air resistance occurs, so that the stable attachment and insertion of the sensor unit may be interfered.

### SUMMARY

An aspect provides an applicator for biometric information acquisition that may maintain an internal space in a sterile, dehumidified, and sealed state.

Another aspect also provides for minimizing and downsizing the packaging to maintain the internal space of the applicator for biometric information acquisition in a sterile, dehumidified, and sealed state.

Another aspect also provides for reducing air resistance generated during the firing process of the applicator for biometric information acquisition.

According to an aspect, there is provided an applicator for biometric information acquisition including a case forming an internal space and including an opening portion opened on one side, a plunger disposed in the internal space of the case and configured to move a sensor unit for biometric information acquisition toward the opening portion, a protection cap configured to shield the opening portion, and a sealing member disposed between the case and the protection cap to seal the internal space.

According to another aspect, there is provided at least one vent configured to communicate with the internal space, wherein at least a portion of the at least one vent is spaced apart from the sealing member.

According to another aspect, there is provided the protection cap configured to surround at least a portion of an outer peripheral surface of the opening portion. The sealing member may be disposed between the outer peripheral surface of the opening portion and the protection cap.

According to another aspect, there is provided at least a portion of the at least one vent formed between the opening portion and the sealing member.

According to another aspect, there is provided the at least one vent configured to provide a path through which air, pushed by the plunger moving toward the opening portion, may be discharged outside the case.

According to another aspect, there is provided the case including a main case forming an outward appearance and an inner case combined to an inner side of the main case and configured to guide the plunger to move toward the opening portion.

According to another aspect, there is provided an end portion of the inner case toward the opening portion bent to surround an end portion of the main case toward the opening portion. The at least one vent may be formed at the inner case.

According to another aspect, there is provided the at least one vent including a first vent and a second vent forming a stepped structure with respect to each other. The second vent may be positioned closer to the opening portion than the first vent and positioned further inside than the first vent.

According to another aspect, there is provided the at least one vent provided as at least a pair of vents, and the pair of vents may be disposed to face each other.

According to another aspect, there is provided the sealing member including an elastic member that is compressed and deformed by the protection cap to fill between the case and the protection cap.

According to another aspect, there is provided the sealing member including a continuously connected ring shape and disposed on a plane intersecting a direction toward the opening portion.

According to another aspect, there is provided the case including a recessed portion formed along a perimeter of the case on an outer peripheral surface of the case and accommodating at least a portion of the sealing member.

According to another aspect, there is provided the sealing member including a band portion accommodated in the recessed portion and at least one raised portion protruding outward from the band portion.

According to another aspect, there is provided the protection cap including a column portion inserted inside the case and an outer wall portion surrounding an outer peripheral surface of the case. The outer wall portion may be in contact with the sealing member on an inner side.

According to another aspect, there is provided the protection cap including a third vent communicating with an inner side of the case, a first cover member configured to seal the third vent, a second cover member configured to seal the first cover member, and a dehumidifying member disposed between the first cover member and the second cover member.

According to another aspect, there is provided the first cover member formed from a material having a bacterial barrier property that prevents bacteria from passing through, and breathability. The second cover member may be formed from a moisture-resistant material.

According to another aspect, there is provided the protection cap including a first accommodating portion inwardly recessed to accommodate the dehumidifying member and a second accommodating portion accommodating the second cover member and forming a stepped structure with the first accommodating portion.

According to another aspect, there is provided the applicator for biometric data acquisition further including an indicator member continuously attached to the protection cap and the case. The indicator member may be configured to be at least partially broken or to leave a mark on the protection cap or the case when the protection cap is removed.

According to another aspect, there is provided the case including a protruding portion provided to reduce a step difference with one surface of the protection cap by protruding outward and to allow the indicator member to be attached.

According to another aspect, there is provided the opening portion formed on an end portion of the case in a first direction. The plunger may be configured to move the sensor unit in the first direction.

According to the example embodiments, it is possible to maintain the internal space of the applicator for biometric information acquisition in a sterile, dehumidified, and sealed state.

According to the example embodiments, it is possible to minimize the packaging and reduce the size of the packaging to maintain the internal space of the applicator for biometric information acquisition in a sterile, dehumidified, and sealed state.

In addition, according to the present disclosure, it is possible to reduce transportation costs for the applicator for biometric information acquisition and to increase efficiency due to the reduced packaging size of the applicator for biometric information acquisition.

Furthermore, according to the present disclosure, it is possible to reduce air resistance that may occur during the firing process of the applicator for biometric information acquisition to stably attach and insert the sensor unit into the body.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is an exploded perspective view illustrating an applicator for biometric information acquisition according to an example embodiment of the present disclosure;
FIG. 2 is an exploded perspective view illustrating a protection cap and an indicator member according to an example embodiment of the present disclosure;
FIG. 3 is a cross-sectional view illustrating an applicator for biometric information acquisition taken along the X-Z plane according to an example embodiment of the present disclosure;
FIG. 4 is an enlarged diagram illustrating an enlarged view of a part A of FIG. 3;
FIG. 5 is a front view illustrating an applicator for biometric information acquisition according to an example embodiment of the present disclosure;
FIG. 6 is a front view illustrating a state in which a protection cap of an applicator for biometric information acquisition is separated according to an example embodiment of the present disclosure;
FIG. 7 is a front view illustrating an applicator for biometric information acquisition to which a different type of indicator member is applied according to example embodiments of the present disclosure;
FIG. 8 is a cross-sectional view illustrating a pre-firing state of an applicator for biometric information acquisition, taken along the X-Z plane, according to an example embodiment of the present disclosure;
FIG. 9 is a cross-sectional view illustrating a post-firing state of an applicator for biometric information acquisition, taken along the X-Z plane, according to an example embodiment of the present disclosure;
FIG. 10 is a cross-sectional view illustrating a state in which a sensor unit is attached and inserted into the skin of a user, taken along the X-Z plane, according to an example embodiment of the present disclosure;
FIG. 11 is a perspective view illustrating a state in which a main case and an inner case are separated according to an example embodiment of the present disclosure;
FIG. 12 is a perspective view illustrating a state in which a main case and an inner case are coupled according to an example embodiment of the present disclosure;
FIG. 13 is a cross-sectional view illustrating a portion of an applicator for biometric information acquisition taken along the Y-Z plane according to an example embodiment of the present disclosure;
FIG. 14 is a cross-sectional view illustrating a pre-firing state of an applicator for biometric information acquisition, taken along the Y-Z plane, according to an example embodiment of the present disclosure; and
FIG. 15 is a cross-sectional view illustrating a post-firing state of an applicator for biometric information acquisition, taken along the Y-Z plane, according to an example embodiment of the present disclosure.

### DETAILED DESCRIPTION

Terms used in example embodiments are selected, as much as possible, from general terms that are widely used at present while taking into consideration the functions of the present disclosure, but these terms may be replaced by other terms based on intentions of those skilled in the art, judicial precedent, the advent of new technologies, or the like. Also, in a particular case, terms that are arbitrarily selected by the applicant of the present disclosure may be used. In this case, the meanings of these terms will be described in detail in corresponding description parts of the disclosure. Accordingly, the terms used herein may be defined not based on simple names, but based on practical meanings thereof and the whole content of the present disclosure.

The suffix "portion" used for elements in this specification is provided or used interchangeably solely for ease of description, and may not have distinct meanings or functions in and of itself. In addition, in describing example embodiments included in the present disclosure, detailed descriptions of related known technologies may be omitted if it is determined that such descriptions may obscure the gist of the embodiments. Furthermore, the accompanying drawings are provided to facilitate understanding of the example embodiments included in the present disclosure and are not intended to limit the technical spirit of the present disclosure, and it should be understood that the present disclosure encompasses all modifications, equivalents, and substitutes within the scope and spirit of the invention.

Terms including ordinal numbers such as "first," "second," and the like may be used to describe various elements, but such elements are not limited by these terms, and these terms may be used merely to distinguish one element from another.

When it is stated that an element is "connected to" or "coupled to" another element, it should be understood that the element may be directly connected to or coupled to the another element, or that another element may exist therebetween. In contrast, when it is stated that an element is "directly connected to" or "directly coupled to" another element, it should be understood that no other elements exist therebetween.

In the description below, a singular expression may include a plural expression unless clearly indicated otherwise in context.

Terms such as "include" or "have" used in this specification should be understood as indicating the existence of a feature, number, step, operation, element, component, or combination thereof described in this specification, and the terms do not exclude in advance the existence or the possibility of one or more additional features, numbers, steps, operations, elements, components, or combinations thereof.

An expression "at least one of a, b, and c" described in this specification may encompass "a alone," "b alone," "c alone," "a and b," "a and c," "b and c," or "all of a, b, and c."

Hereinafter, various example embodiments of the present disclosure will be described in detail with reference to the accompanying drawings for those of ordinary skill in the art to which the present disclosure pertains to easily perform the example embodiments. However, the present disclosure may be embodied in various different forms and not limited to the example embodiments described herein.

Hereinafter, example embodiments of the present disclosure will be described in detail with reference to the accompanying drawings.

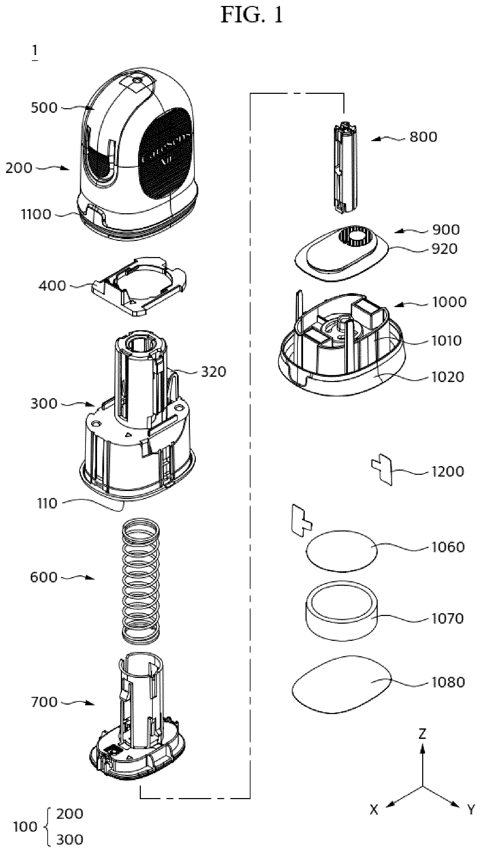

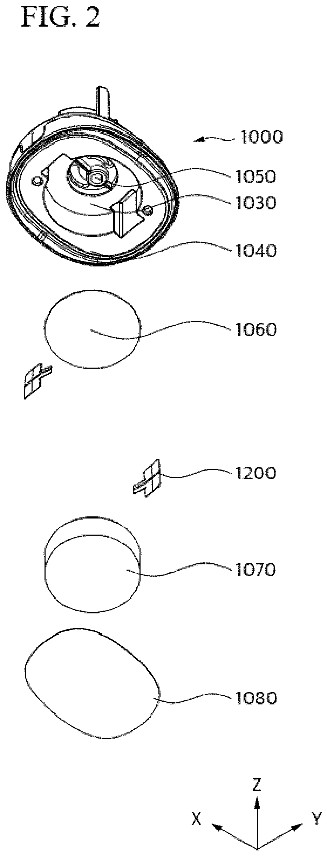

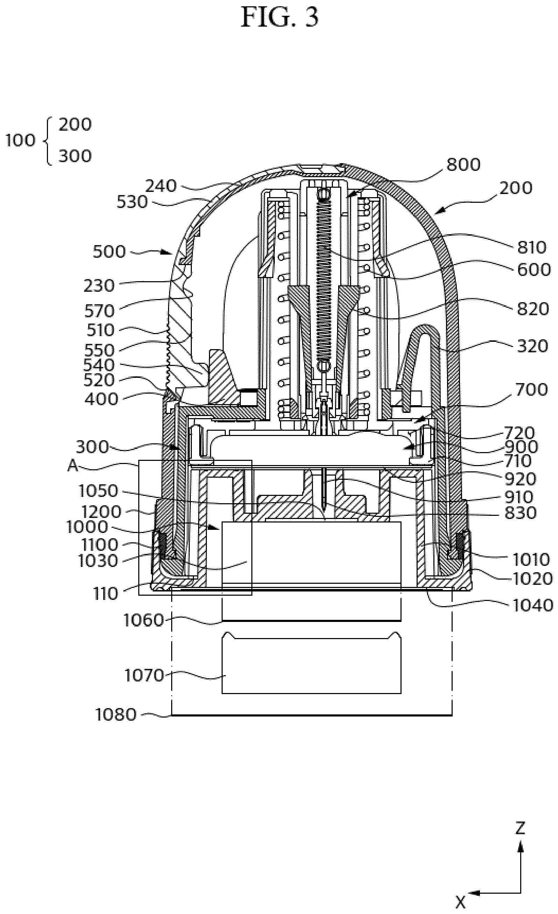

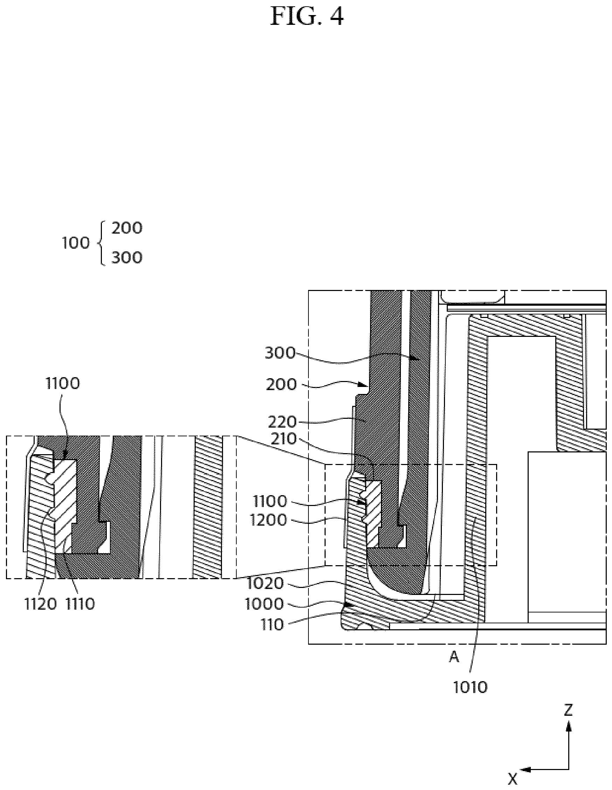

FIG. 1 is an exploded perspective view illustrating an applicator 1 for biometric information acquisition according to an example embodiment of the present disclosure, and FIG. 2 is an exploded perspective view illustrating a protection cap 1000 and an indicator member 1200 according to an example embodiment of the present disclosure, and FIG. 3 is a cross-sectional view illustrating the applicator 1 for biometric information acquisition taken along the X-Z plane according to an example embodiment of the present disclosure, and FIG. 4 is an enlarged diagram illustrating an enlarged view of a part A of FIG. 3.

Referring to FIGS. 1 to 4, the applicator 1 for biometric information acquisition (hereinafter, the applicator 1) according to the present disclosure may include a case 100.

The case 100, according to an example embodiment of the present disclosure, may be a configuration that protects elements of the applicator 1 and forms an outward appearance. The case 100 may form an internal space. The case 100 may form a predetermined space that accommodates other elements inside. At this point, the case 100 may include one open surface. Alternatively, the case 100 may include an opening portion 110. The opening portion 110 may be a portion opened on one side of the case 100. The opening portion 110 may be formed on an end portion in a first direction of the case 100. An opened one surface of the case 100 may be opened in the first direction. The first direction may be a direction that intersects a skin surface. The first direction may be a direction that intersects a skin surface. The first direction described in this specification may be, for example, a direction parallel to a Z axis on a diagram, and more specifically, may be a -Z axis direction on the diagram.

The case 100, according to an example embodiment of the present disclosure, may include a main case 200 and an inner case 300. The main case 200 may be a configuration forming an outward appearance, and the inner case 300 may be a configuration coupled to an inner side of the main case 200. The inner side described herein may refer to an internal space formed by the main case 200. The main case 200 may be a configuration of a dome shape having one surface opened in the first direction, and the inner case 300 may be inserted and assembled to the opened one surface. The inner case 300 may have one surface opened to one side. For example, the inner case 300 may include the aforementioned opening portion 110. As the opening portion 110 may be opened on one side, a sensor unit 900 described later may be inserted through the opening portion 110 and assembled, or discharged during a firing operation.

The case 100, according to an example embodiment of the present disclosure, may include an operating portion 500. The operating portion 500 may be a portion that a user controls to operate the applicator 1. The operating portion 500 may be joined to the case 100 and may seal one opened surface of the case 100. For example, the case 100 may include a button hole 230 formed by penetrating at least a portion of an outer peripheral surface, and the button hole 230 may be formed by penetrating a portion of an outer peripheral surface of the main case 200. At this point, the operating portion 500 may seal the button hole 230.

The operating portion 500, according to an example embodiment of the present disclosure, may include a wrinkled portion 520. The wrinkled portion 520 may be provided to facilitate the movement of a button portion 510 by being elastically deformed.

The operating portion 500, according to an example embodiment of the present disclosure, may include a joint cover 530. The joint cover 530 may provide an area joined to the case 100.

The operating portion 500, according to an example embodiment of the present disclosure, may include a supporting bar 550. The supporting bar 550 may be provided to stably hold a position of a push bar 540.

The supporting bar 550, according to an example embodiment of the present disclosure, may include a bending portion 570. The bending portion 570 may be provided to facilitate movement of the button portion 510 toward an internal space, or the push bar 540 toward the internal space.

The applicator 1, according to an example embodiment of the present disclosure, may include a plunger 700. The plunger 700 may be disposed in the internal space of the case 100. Specifically, the plunger 700 may be disposed in an internal space of the inner case 300. At this point, a first elastic member 600 may be inserted between the plunger 700 and the inner case 300. The first elastic member 600 may be inserted in a compressed state and may expand during firing to move the plunger 700.

The inner case 300, according to an example embodiment of the present disclosure, may be configured to guide the plunger 700 to move toward the opening portion 110. For example, the inner case 300 may guide the plunger 700 to move in the first direction. At least a portion of an inner shape of the inner case 300 may be formed to correspond to a portion of an outward appearance of the plunger 700, and the inner case 300 may include a space in which the plunger 700 moves toward the opening portion 110. The plunger 700 may move toward the opening portion 110 as being guided by at least a portion of an inner surface of the inner case 300.

The plunger 700, according to an example embodiment of the present disclosure, may be configured to move the sensor unit 900 toward the opening portion 110. For example, the sensor unit 900 may move to the first direction. The sensor unit 900 may be a configuration to obtain biometric information of the user. The plunger 700 may move toward the opening portion 110 by the first elastic member 600, and at this point, move toward the opening portion 110 together with the sensor unit 900.

The applicator 1, according to an example embodiment of the present disclosure, may further include a trigger 400. The trigger 400 may maintain a stationary state of the plunger 700 and release the stationary state of the plunger 700 during movement. The user may move the trigger 400 by pressing the button portion 510 of the operating portion 500 described above.

The applicator 1, according to an example embodiment of the present disclosure, may include a needle assembly 800. The needle assembly 800 may move a needle 830 toward the opening portion during firing, and a moved needle 830 may penetrate a portion of a skin S of the user. The needle assembly 800 may move the needle 830 again in a reverse direction at a predetermined point after firing and retrieve the needle 830. For example, the needle 830 may move in the first direction to penetrate the skin S of the user, and move in a second direction, which is a reverse direction of the first direction, to be retrieved. The second direction described in this specification may be, for example, a direction parallel to the Z axis on a diagram, and more specifically, may be a +Z axis direction on the diagram.

In this specification, a direction in which the plunger 700, the sensor unit 900, the needle assembly 800, and the needle 830 moves is described as the first direction, but it is merely an example, and a moving direction is not limited to the first direction and may be properly modified if the needle 830 may penetrate the skin S of the user, and the sensor unit 900 may be attached to the skin S of the user. For example, although the opening portion 110 is opened to the first direction, the needle 830 may be propelled and moved in a direction intersecting the first direction and may penetrate a portion of the skin S of the user. In the same manner, a direction in which a protection cap 1000 described later is coupled to or separated from the case 100 may intersect with a direction in which the needle 830 moves.

The needle assembly 800, according to an example embodiment of the present disclosure, may be inserted inside the plunger 700. Specifically, the needle assembly 800 may be accommodated within a protruding space on an upper portion of the plunger 700, and the sensor unit 900 may be disposed below the needle assembly 800.

The applicator 1, according to an example embodiment of the present disclosure, may include the protection cap 1000. The protection cap 1000 may be configured to shield the opening portion 110 of the case 100. The protection cap 1000 may be configured to surround at least a portion of an outer peripheral surface of the opening portion 110 of the case 100. For example, the protection cap 1000 may be formed to surround at least a portion of an outer peripheral surface of an end portion positioned in the first direction among end portions of the case 100. The protection cap 1000 may include a concave cap shape to allow the opening portion 110 of the case 100 to be inserted. At least a portion of the opening portion 110 of the case 100 may be inserted into the protection cap 1000.

The protection cap 1000, according to an example embodiment of the present disclosure, may include a column portion 1010 and an outer wall portion 1020. The column portion 1010 may be a portion that is inserted inside the case 100, and the outer wall portion 1020 may be a portion that surrounds an outer peripheral surface of the case 100. The column portion 1010 may surround the inside of the opening portion 110, and the outer wall portion 1020 may surround the outside of the opening portion 110. For example, a groove may be formed between the column portion 1010 and the outer wall portion 1020 of the protection cap 1000 to allow an end portion in the first direction of the case 100 to be inserted. The column portion 1010 may surround the inside of the end portion in the first direction of the case 100, and the outer wall portion 1020 may surround the outside of the end portion in the first direction of the case 100 to shield one opened surface of the case 100.

The applicator 1, according to an example embodiment of the present disclosure, may include a sealing member 1100. The sealing member 1100 may be configured to be disposed between the case 100 and the protection cap 1000 to seal the internal space of the case 100. For example, the sealing member 1100 may fill an empty space that may be formed between the protection cap 1000 and the case 100. Specifically, the outer wall portion 1020 of the protection cap 1000 may be in contact with the sealing member 1100 inwardly. The sealing member 1100 may be disposed between the outer peripheral surface of the opening portion 110 of the case 100 and the protection cap 1000. The sealing member 1100 may fill an empty space between the main case 200 and the outer wall portion 1020 at a portion adjacent to the opening portion 110.

The sealing member 1100, according to an example embodiment of the present disclosure, may include an elastic material. The sealing member 1100 may be compressed and deformed by the protection cap 1000 to fill between the case 100 and the protection cap 1000. For example, an elastic material such as rubber may be applied.

The sealing member 1100, according to an example embodiment of the present disclosure, may include a continuously connected ring shape. A ring shape of the sealing member 1100 may be disposed to surround at least a portion of the outer peripheral surface of the case 100.

The sealing member 1100, according to an example embodiment of the present disclosure, may include a band portion 1110 and at least one raised portion 1120. Referring to FIG. 4, the band portion 1110 may be a portion of a relatively thick ring shape, and the raised portion 1120 may be a portion protruding outward from the band portion 1110. The case 100 may include a recessed portion 210. The recessed portion 210 may be formed along a perimeter of the case 100 on the outer peripheral surface of the case 100 and may accommodate at least a portion of the sealing member 1100. The band portion 1110 of the sealing member 1100 may be accommodated in the recessed portion 210. The band portion 1110 may be attached to the case 100 by being accommodated in the recessed portion 210. The raised portion 1120 may fill an empty space between the protection cap 1000 and the case 100 by protruding outward from the band portion 1110. A plurality of raised portions 1120 may be formed, and the sealing strength may be adjusted by varying the number of the raised portions 1120.

The protection cap 1000, according to an example embodiment of the present disclosure, may be coupled to or separated from the case 100 in a direction toward the opening portion 110 and the opposite direction. For example, the protection cap 1000 may be coupled to or separated from the case 100 in the first direction and the second direction. The sealing member 1100 may be disposed on a plane intersecting a direction toward the opening portion 110. The sealing member 1100 may be spaced apart from the opening portion 110 by a predetermined interval. The sealing member 1100 may provide uniform sealing strength between the protection cap 1000 and the case 100.

The operating portion 500, according to an example embodiment of the present disclosure, may include a rubber material to be operated by the user, and the operating portion 500 may be joined to the case 100 to seal the button hole 230 of the case 100. In addition, the sealing member 1100 may be joined to the case 100 to seal between the protection cap 1000 and the case 100. The operating portion 500 and the sealing member 1100 may be formed from the same material and formed from a different material from the case 100. At this point, the operating portion 500 and the sealing member 1100 may be formed together with the case 100. For example, the operating portion 500 and the sealing member 1100 may be manufactured together with the case 100 in a double injection molding method. However, such a manufacturing method is merely an example, and a manufacturing method in which the operating portion 500 and the sealing member 1100 are bonded to the case 100 by applying an adhesive to the case 100.

The protection cap 1000, according to an example embodiment of the present disclosure, may include a third vent 1050. The third vent 1050 may be a hole communicating with the inside of the case 100. In a state in which the protection cap 1000 is coupled to the case 100, an internal space of the case 100 may be sealed except for the third vent 1050. At this point, a sterile gas may be injected through the third vent 1050. The injected sterile gas may sterilize the internal space of the case 100.

The protection cap 1000, according to an example embodiment of the present disclosure, may include a first cover member 1060. The first cover member 1060 may be configured to seal the third vent 1050. The first cover member 1060 may cover and seal the outer surface of the third vent 1050.

The protection cap 1000, according to an example embodiment of the present disclosure, may include a second cover member 1080. The second cover member 1080 may be configured to seal the first cover member 1060. The second cover member 1080 may cover and seal the outside of the first cover member 1060.

The protection cap 1000, according to an example embodiment of the present disclosure, may include a dehumidifying member 1070. The dehumidifying member 1070 may be a configuration to remove humidity. The dehumidifying member 1070 may be disposed between the first cover member 1060 and the second cover member 1080.

The third vent 1050, the first cover member 1060, the dehumidifying member 1070, and the second cover member 1080 according to an example embodiment of the present disclosure may be disposed sequentially in one direction. For example, the third vent 1050, the first cover member 1060, the dehumidifying member 1070, and the second cover member 1080 may be sequentially disposed along the first direction.

The first cover member 1060 and the second cover member 1080 according to an example embodiment of the present disclosure may be formed from different materials from each other.

The first cover member 1060 according to an example embodiment of the present disclosure may be formed from a material having a bacterial barrier property that does not allow bacteria to pass through and breathability. The first cover member 1060 may include a porous filter structure. Accordingly, when the sterile gas is injected even in a state in which the first cover member 1060 seals the third vent 1050, the sterile gas may be injected into the internal space of the case 100 by passing through the first cover member 1060 and through the third vent 1050. However, the porous filter structure of the first cover member 1060 may be formed small enough to prevent bacteria from passing through. Accordingly, external bacteria may not pass through the first cover member 1060.

The second cover member 1080 according to an example embodiment of the present disclosure may be formed from a moisture-resistant material. For example, the second cover member 1080 may be an aluminum film. Accordingly, external moisture may not pass through the second cover member 1080.

Referring to FIG. 2, the first cover member 1060, the dehumidifying member 1070, and the second cover member 1080 according to an example embodiment of the present disclosure may be accommodated in the protection cap 1000 as a triple-layer structure and may maintain the internal environment of the case 100 in a sterile, dehumidified, and sealed state. After sterilizing the internal space of the case 100 by injecting the sterile gas in a state in which the first cover member 1060 covers the third vent 1050, the dehumidifying member 1070 may be disposed, and the second cover member 1080 may be covered the outside to form a structure that prevents external bacteria and moisture from penetrating into the internal space of the case 100.

The protection cap 1000, according to an example embodiment of the present disclosure, may include a first accommodating portion 1030. The first accommodating portion 1030 may be a portion of the protection cap 1000 that is inwardly recessed to accommodate the dehumidifying member 1070. Specifically, the first accommodating portion 1030 may be a space formed by being inwardly recessed from an end portion in the first direction of the column portion 1010. The third vent 1050 may be formed by penetrating the top surface of the first accommodating portion 1030. The first cover member 1060 may cover the top surface of the first accommodating portion 1030. Further below, the dehumidifying member 1070 may be disposed in the remaining space of the first accommodating portion 1030.

The protection cap 1000, according to an example embodiment of the present disclosure, may include a second accommodating portion 1040. The second accommodating portion 1040 may be a portion of the protection cap 1000 in which the second cover member 1080 is accommodated. The second accommodating portion 1040 may be formed wider and thinner than the first accommodating portion 1030. The second cover member 1080 may be formed wider than the first cover member 1060 and the dehumidifying member 1070. The second accommodating portion 1040 and the first accommodating portion 1030 may form a stepped structure with respect to each other. Referring to FIG. 4, the second accommodating portion 1040 may be disposed below the first accommodating portion 1030 and formed wider than the first accommodating portion 1030 in a direction perpendicular to the first direction. For example, the direction perpendicular to the first direction may be a third direction. The third direction may be a direction parallel to an X axis direction or a Y axis direction on the diagram.

As the protection cap 1000 according to an example embodiment of the present disclosure may accommodate the first cover member 1060, the dehumidifying member 1070, and the second cover member 1080 inside, the protection cap 1000 may simplify the packaging for sterilization, dehumidification, and sealing of the applicator 1, and the size of the packaging may also decrease.

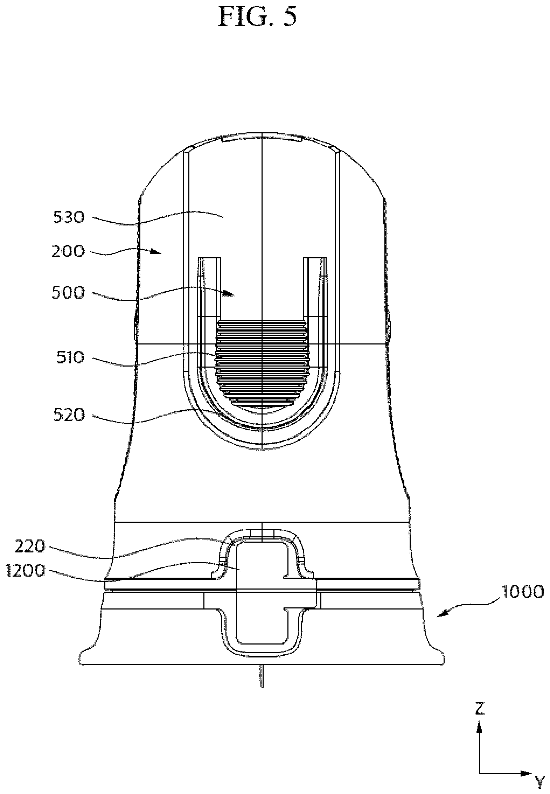

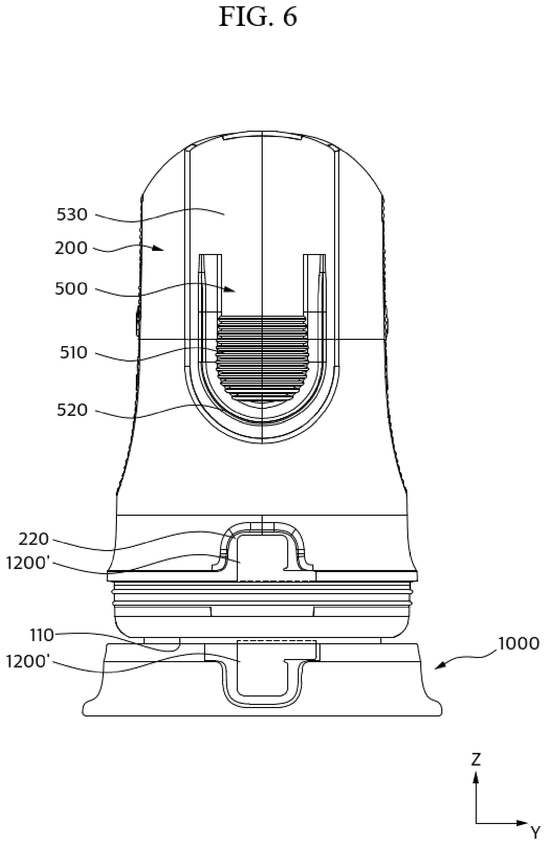

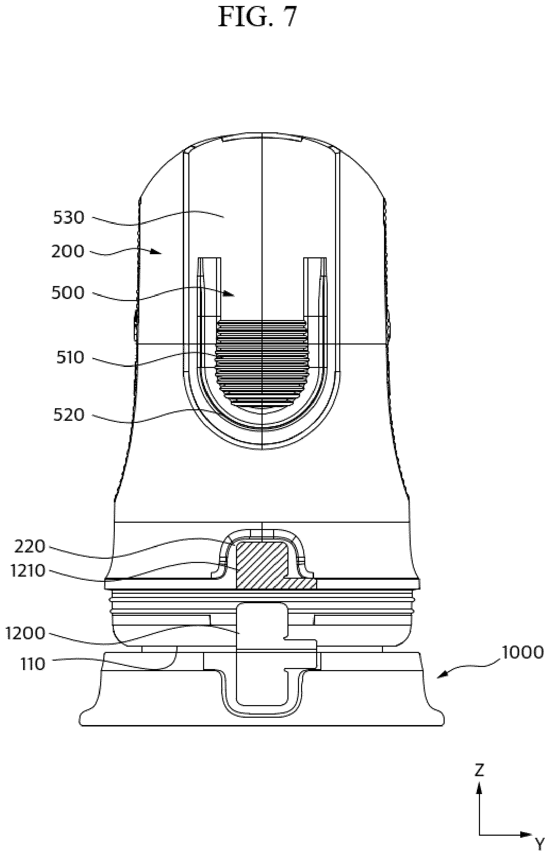

FIG. 5 is a front view illustrating the applicator 1 for biometric information acquisition according to an example embodiment of the present disclosure, and FIG. 6 is a front view illustrating a state in which the protection cap 1000 of the applicator 1 for biometric information acquisition is separated according to an example embodiment of the present disclosure, and FIG. 7 is a front view illustrating the applicator 1 for biometric information acquisition to which a different type of the indicator member 1200 is applied according to example embodiments of the present disclosure.

Referring to FIGS. 5 to 7, the applicator 1 may further include the indicator member 1200.

Until the applicator 1 according to an example embodiment of the present disclosure is used, the internal space of the applicator 1 may be maintained in a sterile, dehumidified, and sealed state. When the protection cap 1000 of the applicator 1 is removed from the case 100, the sterile, dehumidified, and sealed state of the internal space of the case 100 may be compromised, and the applicator 1 may not be reused.

The indicator member 1200, according to an example embodiment of the present disclosure, may be a configuration to identify whether such a sterile, dehumidified, and sealed state is compromised or not and may be provided to leave a mark when the protection cap 1000 is once removed.

The indicator member 1200, according to an example embodiment of the present disclosure, may be continuously attached across the protection cap 1000 and the case 100. A single indicator member 1200 may be continuously attached across a portion of the outer surface of the protection cap 1000 and a portion of the outer surface of the case 100. The indicator member 1200 may be attached to a connection point of the protection cap 1000 and the case 100.

Specifically, a portion of the indicator member 1200, according to an example embodiment of the present disclosure, may be attached one surface of the outer wall portion 1020 of the protection cap 1000, and the remaining portion may be attached to the protruding portion 220 of the case 100. The case 100 may include the protruding portion 220. The protruding portion 220 may be a configuration to which the indicator member 1200 is attached, and as the protruding portion 220 protrudes outward from the case 100, the step difference between one surface of the protection cap 1000 and the outer surface of the case 100 may be reduced. Therefore, the indicator member 1200 may be attached to the outer surface of the protection cap 1000 and the outer surface of the case 100 without excessive bending.

The indicator member 1200, according to an example embodiment of the present disclosure, may be provided to be at least partially broken, or to leave a mark 1210 on the protection cap 1000 or the case 100 when the protection cap 1000 is removed.

Referring to FIG. 6, the indicator member 1200, according to an example embodiment of the present disclosure, may include a partially broken breaking line to facilitate breakage. When the protection cap 1000 is removed, the indicator member 1200 may be broken along the breaking line. A portion of a broken indicator member 1200' may be attached to the protection cap 1000, and the remaining portion may be attached to the case 100.

Referring to FIG. 7, the indicator member 1200 according to another example embodiment of the present disclosure may be configured to leave the mark 1210 such as an ink mark or adhesive on an object when removed from an attached object. For example, when the protection cap 1000 is removed, the indicator member 1200 may be attached to the protection cap 1000, and the mark 1210 such as an ink mark may remain on the protruding portion 220 to which the indicator member 1200 is attached.

FIGS. 6 and 7 may be merely examples among various ways in which the indicator member 1200 leaves a mark, and the ways may be appropriately modified within the scope of achieving the goal of the present disclosure.

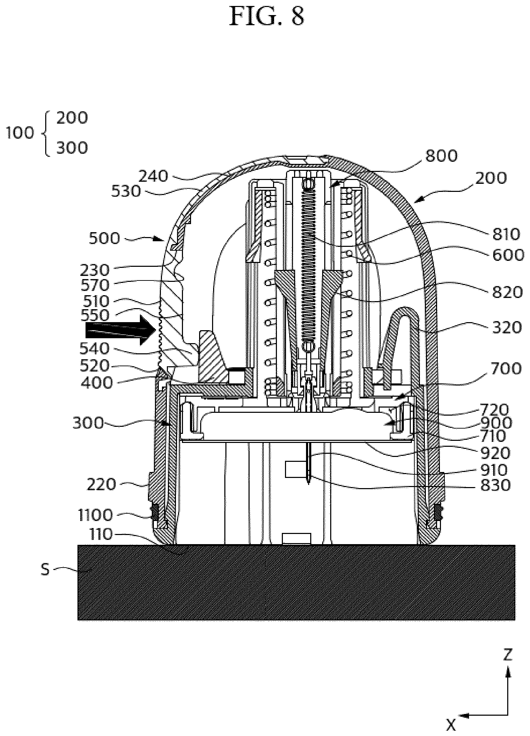

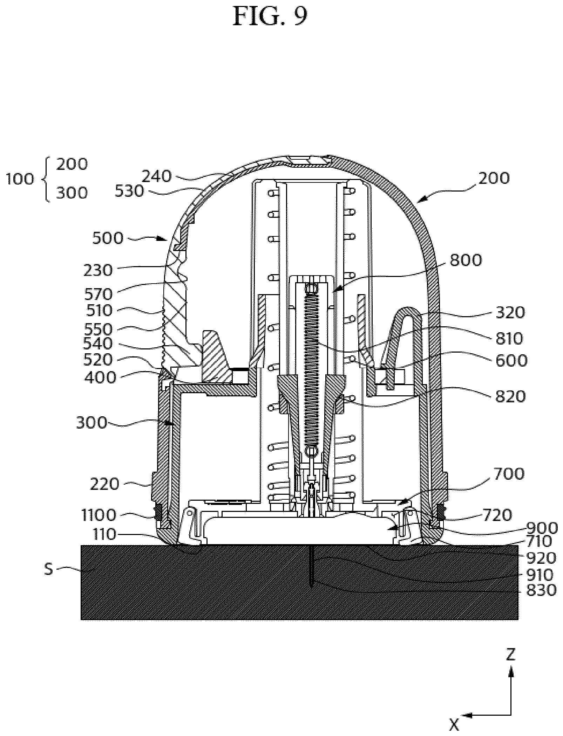

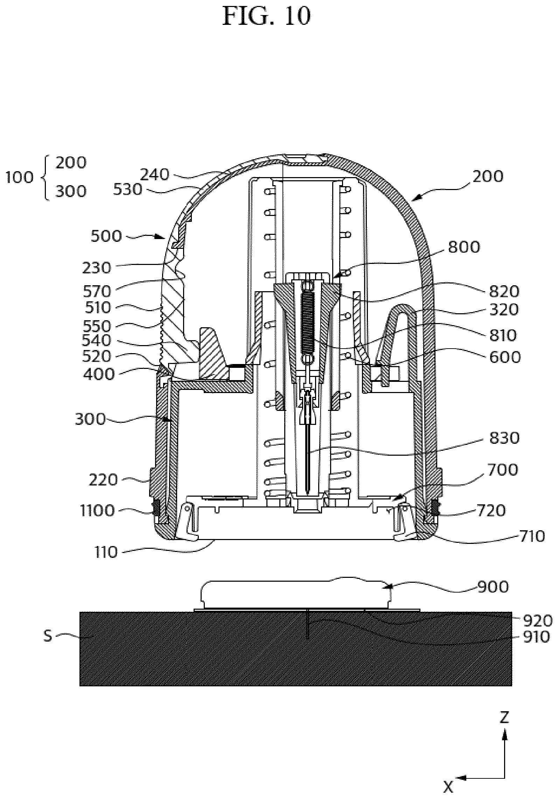

FIG. 8 is a cross-sectional view illustrating a pre-firing state of the applicator 1 for biometric information acquisition, taken along the X-Z plane, according to an example embodiment of the present disclosure, and FIG. 9 is a cross-sectional view illustrating a post-firing state of the applicator 1 for biometric information acquisition, taken along the X-Z plane, according to an example embodiment of the present disclosure, and FIG. 10 is a cross-sectional view illustrating a state in which the sensor unit 900 is attached and inserted into the skin S of a user, taken along the X-Z plane, according to an example embodiment of the present disclosure.

Referring to FIGS. 8 and 9, firing mechanism of the applicator 1 will be described.

FIG. 8 illustrates the applicator 1 in a pre-firing state, and referring to FIG. 8, the user may remove the protection cap 1000 to use the applicator 1 and position the applicator 1 so that the opening portion 110 of the case 100 comes into contact with the skin S of the user. For example, an end portion of the case 100 in the first direction may be in contact with the skin S of the user.

The plunger 700, according to an example embodiment of the present disclosure, may hold the sensor unit 900. A socket 720 may be on a bottom of a direction toward the opening portion 110 of the plunger 700, and the socket 720 may form a predetermined space to accommodate the sensor unit 900. At this point, at least one hook 710 may be formed around the socket 720. One end of the hook 710 may be bent, and the band portion of the hook 710 may support at least a portion of the sensor unit 900. The sensor unit 900 supported by the hook 710 may remain fixed within the socket 720.

The plunger 700, according to an example embodiment of the present disclosure, may be in a stationary state at the mid-height or the center of the case 100. At this point, a first elastic member 600 may be compressed between the plunger 700 and the inner case 300. Before firing, the trigger 400 may be positioned relatively on the left side in the diagram and may be engaged with at least a portion of the plunger 700 and may fix the plunger 700 so that the plunger 700 is not allowed to move by the first elastic member 600. In other words, the trigger 400 may maintain the plunger 700 in a stationary state.

The sensor unit 900, according to an example embodiment of the present disclosure, may include a probe 910 inserted into a body, and the probe 910 may protrude to one side. For example, the probe 910 may protrude in a direction toward the opening portion 110 in a pre-firing state. An adhesive layer 920 may be formed on the bottom of the body of the sensor unit 900, and the adhesive layer 920 may be formed wider than the sensor unit 900. The adhesive layer 920 may attach to the body, and the sensor unit 900 may be fixed to the skin S.

The needle assembly 800, according to an example embodiment of the present disclosure, may be accommodated in the top portion of the plunger 700. One end of the needle 930 may be sharp and may penetrate the skin S, and may accommodate the probe 910 by forming an empty space inside.

The user may position the applicator 1 on a using location and fire the applicator 1 by pressing the button portion 510.

FIG. 9 illustrates the post-firing state, and referring to FIG. 9, the plunger 700 may move toward the opening portion 110 and attach the sensor unit 900 to the skin S.

The user may push the button portion 510 toward the internal space of the case 100. At least a portion of the button portion 510 may be pushed inside the button hole 230. At this point, the push bar 540 positioned inside the button portion 510 may be moved into the internal space. One end of the push bar 540 may pressure the trigger 400, and the trigger 400 may move into the internal space. One surface of the trigger 400 may be in contact with the push bar 540, and an opposite surface may be in contact with a return member 320. The return member 320 may be a configuration provided in the inner case 300 and elastically deformed to move the trigger 400 to the original position. For example, when the user releases the button portion 510, the trigger 400 may return to the original position by the return member 320.

The trigger 400 moved to the internal space may release the stationary state of the plunger 700. The plunger 700 may move toward the opening portion 110 by the expansion of the compressed first elastic member 600. For example, the plunger 700 may move to the first direction.

The sensor unit 900 and the needle assembly 800, according to an example embodiment of the present disclosure, may move toward the opening portion 110 together with the plunger 700.

The needle 830 of the needle assembly 800, according to an example embodiment of the present disclosure, may move toward the opening portion 110 and penetrate the skin S by a predetermined depth. The probe 910 positioned inside the needle 830 may be inserted into a hole of the skin S formed by the needle 830. The adhesive layer 920 of the sensor unit 900 may be attached to the surface of the skin S, and the sensor unit 900 may be fixed to the skin S in a state in which the probe 910 is inserted into the skin S.

The inner case 300, according to an example embodiment of the present disclosure, may include a guide wall on the inside wall, and the guide wall may guide the movement of the hook 710 along the moving path of the hook 710. The guide wall may widen outward toward the opening portion 110. For example, the guide wall may widen outward toward the first direction. One surface of the hook 710 may move in the first direction while remaining in contact with the guide wall. The hook 710 may rotate upon reaching the end portion in the first direction. Due to the rotation of the hook 710, the band portion of the hook 710 that supports the bottom of the sensor unit 900 may be apart from the sensor unit 900, and the hook 710 may release the fixation of the sensor unit 900.

FIG. 10 illustrates a state in which the sensor unit 900 is attached to the skin S. Referring to FIGS. 9 and 10, the needle 830 may be retrieved after the sensor unit 900 is attached to the skin S.

The needle assembly 800, according to an example embodiment of the present disclosure, may include a retrieval portion 820. One end of the retrieval portion 820 may fix the needle 830. The retrieval portion 820 may be hooked and fixed on a portion of the frame of the needle assembly 800. At a point when the needle 830 penetrates the skin S by a predetermined depth as the needle assembly 800 moves toward the opening portion 110, the retrieval portion 820 may be pressed to an inward direction by the inner case 300. At this point, the retrieval portion 820 may be disengaged from the frame of the needle assembly 800.

The needle assembly 800, according to an example embodiment of the present disclosure, may include a second elastic member 810. The second elastic member 810 may be coupled to the retrieval portion 820 in a tensioned state. When the retrieval portion 820 is disengaged from the frame of the needle assembly 800, the second elastic member 810 may be pulled in a direction (for example, the second direction) opposite to the direction in which the retrieval portion 820 is moved.

The needle 830 may be retrieved by the retrieval portion 820 according to an example embodiment of the present disclosure in a direction (for example, the second direction) opposite to the direction in which the needle 830 is moved, and accommodated within the needle assembly 800. The retrieved needle 830 may not be exposed outward so that the user may not be injured. As described above, it is merely an example that the moving direction of the needle assembly 800 and the needle 830 is described as the first direction, and the moving direction of the needle assembly 800 and the needle 830 may be a direction intersecting with the first direction.

The mechanism and configurations with regard to firing of the applicator 1 described in FIGS. 8 to 10 may be merely an example. Accordingly, even when a different firing mechanism is applied to the applicator 1, a configuration for maintaining sterilization, dehumidification, and sealing may be applied in the same manner, and may be applied identically to a vent 310 configuration described later.

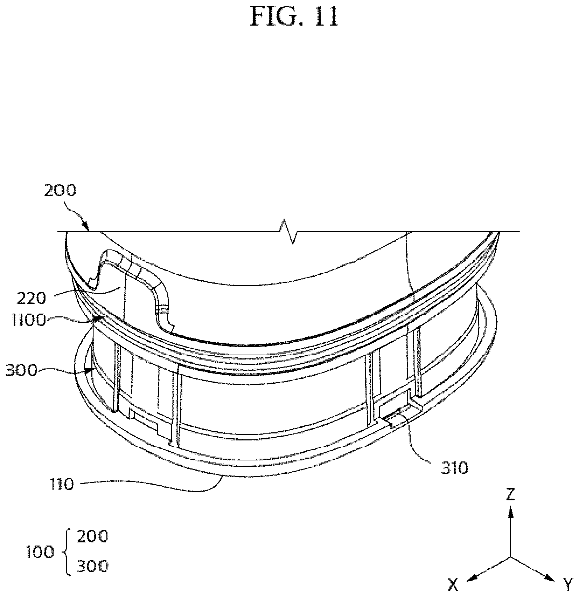

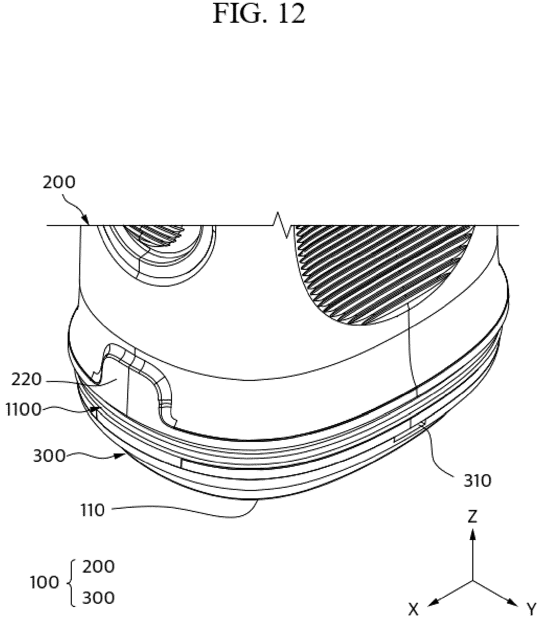

FIG. 11 is a perspective view illustrating a state in which the main case 200 and the inner case 300 are separated according to an example embodiment of the present disclosure, and FIG. 12 is a perspective view illustrating a state in which the main case 200 and the inner case 300 are coupled according to an example embodiment of the present disclosure.

Referring to FIGS. 11 to 12, the applicator 1 may further include at least one vent 310. The vent 310 may be a hole penetrating the case 100 and communicating with the internal space of the case 100. The vent 310 may be a hole configured to discharge air in the internal space of the case 100 to the outside. The vent 310 may be empty inside to allow air to enter and exit smoothly.

The vent 310, according to an example embodiment of the present disclosure, may be formed at the inner case 300. An end portion of the inner case 300 toward the opening portion 110 may be bent, and the band portion of the inner case 300 may surround an end portion toward the opening portion 110 of the main case 200. For example, the end portion of the inner case 300 in the first direction may be positioned lower than the end portion of the main case 200 in the first direction.

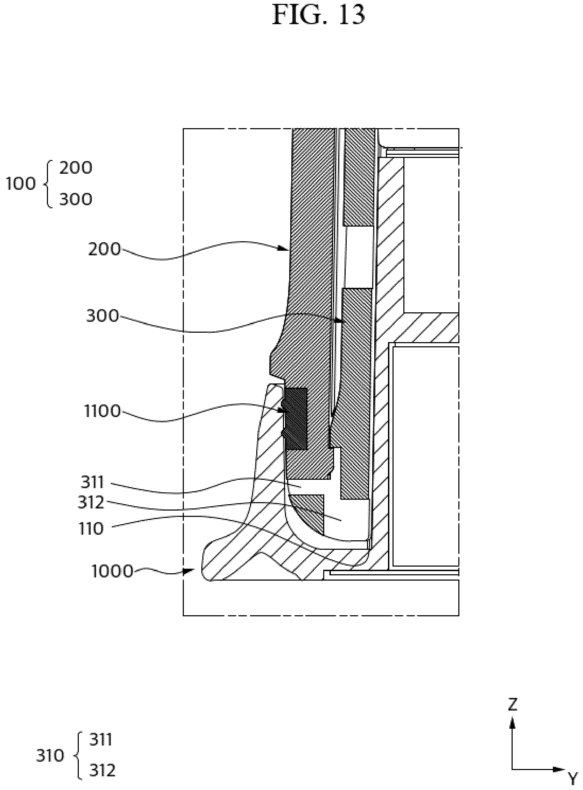

The vent 310 formed on the inner case 300 may be formed in a stepped structure. For example, referring to FIG. 13 together, the vent 310 may include a first vent 311 and a second vent 312. The first vent 311 may be positioned relatively outside with respect to the case 100, and the second vent 312 may be positioned relatively inside. The first vent 311 may be positioned at a higher position than the second vent 312. For example, the first vent 311 may be positioned above to be spaced apart from the opening portion 110. Referring to FIG. 11, before the inner case 300 and the main case 200 are coupled, at least a portion of the vent 310 may include a form opened upward. Specifically, at least a portion of the first vent 311 may be opened upward. Referring to FIG. 12, after the inner case 300 and the main case 200 are coupled, a portion of the main case 200 may cover an opened upper portion of the first vent 311. Detailed description about the first vent 311 and the second vent 312 will be described later.

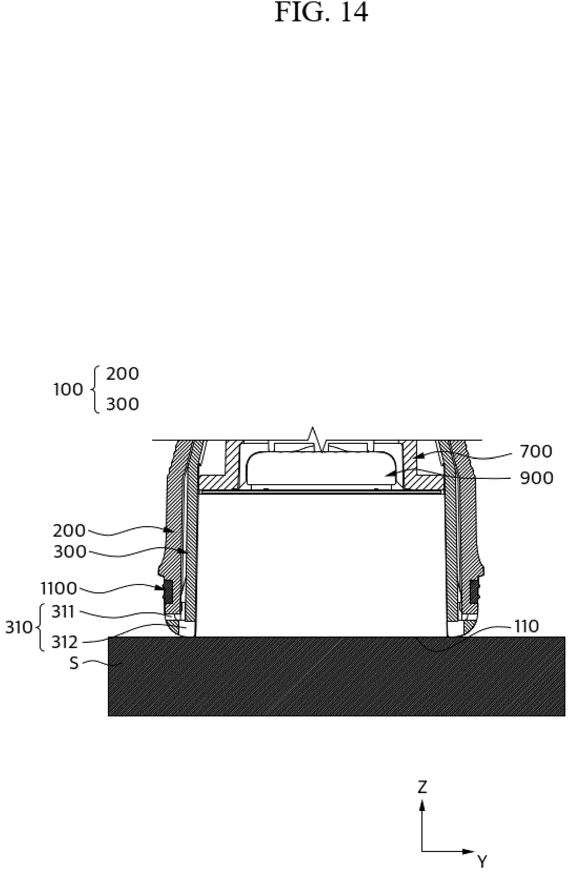

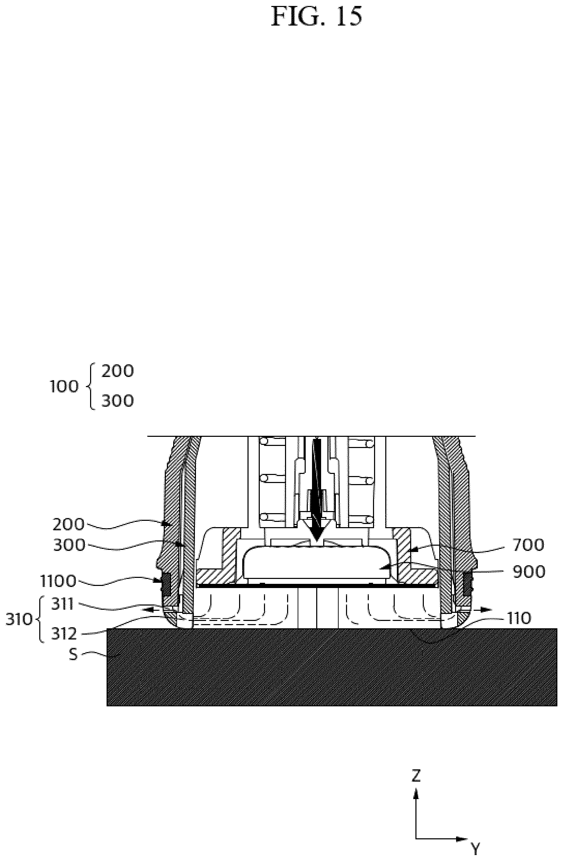

FIG. 13 is a cross-sectional view illustrating a portion of the applicator 1 for biometric information acquisition taken along the Y-Z plane according to an example embodiment of the present disclosure, and FIG. 14 is a cross-sectional view illustrating a pre-firing state of the applicator 1 for biometric information acquisition, taken along the Y-Z plane, according to an example embodiment of the present disclosure, and FIG. 15 is a cross-sectional view illustrating a post-firing state of the applicator 1 for biometric information acquisition, taken along the Y-Z plane, according to an example embodiment of the present disclosure.

Referring to FIGS. 13 to 15, the vent 310, according to an example embodiment of the present disclosure, may be configured to provide a path for discharging the air in the internal space of the case 100 to the outside. Specifically, the vent 310 may provide a path for discharging the air pushed by the plunger 700 that moves toward the opening portion 110 during firing to the outside.

The vent 310, according to an example embodiment of the present disclosure, may be spaced apart from the sealing member 1100 toward the opening portion 110. For example, the vent 310 may be spaced apart further in the first direction than the sealing member 1100. The vent 310 may be a hole configured to discharge the air in the internal space of the case 100 to the outside, and may communicate with the outside. Until the applicator 1 is used, the internal space of the case 100 may be maintained sealed, and the vent 310 may also be sealed. Accordingly, when the protection cap 1000 is coupled to the case 100, the vent 310 may be positioned further in the first direction than the sealing member 1100 so that the vent may not communicate with the outside. The sealing member 1100 may seal between the protection cap 1000 and the case 100, and the vent 310 positioned further in the first direction than the sealing member 1100 may be sealed.

The first vent 311 and the second vent 312 according to an example embodiment of the present disclosure may form a stepped structure with respect to each other. The first vent 311 and the second vent 312 may form a step difference. The first vent 311 and the second vent 312 may form the step difference toward the opening portion 110. For example, the first vent 311 and the second vent 312 may form the step difference in the first direction. The second vent 312 may be positioned closer to the opening portion 110 and further inside than the first vent 311. During use, at least one surface of the second vent 312 may be in contact with the skin S of the user and positioned at an end portion of the case 100 in the first direction. At least a portion of the second vent 312 may include a shape opened downward. Accordingly, the air pushed by the plunger 700 that moves in the first direction may be discharged to the outside through the vent 310 until the end of the firing.

At least a portion of the vent 310, according to an example embodiment of the present disclosure, may be formed between the opening portion 110 of the case 100 and the sealing member 1100. For example, the first vent 311 may be positioned between an end portion in the first direction and the sealing member 1100. The first vent 311 may be formed to be spaced apart from the end portion of the case 100 in the first direction and may prevent the stiffness of the end portion of the case 100 in the first direction from being reduced.

The first vent 311, according to an example embodiment of the present disclosure, may be a hole toward the outside of the case 100. For example, the first vent 311 may include a shape opened in a radial direction with respect to the longitudinal direction of the case 100 when the main case 200 and the inner case 300 are coupled. For example, the first vent 311 may be opened in a direction parallel to the third direction on a diagram. At this point, the second vent 312 may also be opened in the direction parallel to the third direction.

The first vent 311 and the second vent 312, according to an example embodiment of the present disclosure, may be connected to each other. For example, the first vent 311 and the second vent 312 may be connected to each other in a stepped configuration. At least a portion of the first vent 311 may be positioned above at least a portion of the second vent 312 to be in communication with each other.

According to an example embodiment of the present disclosure, a plurality of vents 310 may be formed. For example, at least a pair of vents 310 may be formed. At least a pair of vents 310 may be disposed to face each other. The air in the internal space of the case 100 may be discharged in a balanced manner through at least a pair of vents 310.

The shape, number, and position of the aforementioned vent 310 are merely an example. Accordingly, the first vent 311 and the second vent 312 may be formed to penetrate along a straight line or a diagonal line, and the vent 310 are not necessarily provided in pairs, but may be formed in an odd number.

While the present disclosure is illustrated and described with reference to desired example embodiments to exemplify the principle of the present disclosure, the present disclosure is not limited to the configuration and operation exactly as shown and described. Rather, it will be understood by those skilled in the art that various modifications and alterations may be made to the present disclosure without departing from the spirit and scope of the appended claims.

## Claims

1. An applicator for biometric information acquisition, comprising:
a case forming an internal space and including an opening portion opened on one side,
a plunger disposed in the internal space of the case and configured to move a sensor unit for biometric information acquisition toward the opening portion;
a protection cap configured to shield the opening portion;
a sealing member disposed between the case and the protection cap to seal the internal space; and
at least one vent configured to communicate with the internal space,
wherein at least a portion of the at least one vent is spaced apart from the sealing member.

2. The applicator for biometric information acquisition of claim 1,
wherein the protection cap is configured to surround at least a portion of an outer peripheral surface of the opening portion, and
wherein the sealing member is disposed between the outer peripheral surface of the opening portion and the protection cap.

3. The applicator for biometric information acquisition of claim 1 or 2,
wherein at least a portion of the at least one vent is formed between the opening portion and the sealing member.

4. The applicator for biometric information acquisition of any one of the preceding claims,
wherein the at least one vent is configured to provide a path through which air, pushed by the plunger moving toward the opening portion, is discharged outside the case.

5. The applicator for biometric information acquisition of any one of the preceding claims,
wherein the case comprises:
a main case forming an outward appearance; and
an inner case combined to an inner side of the main case and configured to guide the plunger to move toward the opening portion.

6. The applicator for biometric information acquisition of claim 5,
wherein an end portion of the inner case toward the opening portion is bent to surround an end portion of the main case toward the opening portion,
wherein the at least one vent is formed at the inner case,
wherein the at least one vent includes a first vent and a second vent forming a stepped structure with respect to each other, and
wherein the second vent is positioned closer to the opening portion than the first vent and positioned further inside than the first vent.

7. The applicator for biometric information acquisition of any one of the preceding claims,
wherein the at least one vent is provided as at least a pair of vents, and the pair of vents is disposed to face each other,
wherein the opening portion is formed on an end portion of the case in a first direction, and
wherein the plunger is configured to move the sensor unit in the first direction.

8. The applicator for biometric information acquisition of any one of the preceding claims,
wherein the sealing member includes an elastic material that is compressed and deformed by the protection cap to fill between the case and the protection cap, and
wherein the sealing member includes a continuously connected ring shape and is disposed on a plane intersecting a direction toward the opening portion.

9. The applicator for biometric information acquisition of any one of the preceding claims,
wherein the case includes a recessed portion formed along a perimeter of the case on an outer peripheral surface of the case and accommodating at least a portion of the sealing member, and
wherein the sealing member includes a band portion accommodated in the recessed portion and at least one raised portion protruding outward from the band portion.

10. The applicator for biometric information acquisition of any one of the preceding claims,
wherein the protection cap includes a column portion inserted inside the case and an outer wall portion surrounding an outer peripheral surface of the case, and
wherein the outer wall portion is in contact with the sealing member on an inner side.

11. The applicator for biometric information acquisition of any one of the preceding claims,
wherein the protection cap comprises:
a third vent communicating with an inner side of the case;
a first cover member configured to seal the third vent;
a second cover member configured to seal the first cover member; and
a dehumidifying member disposed between the first cover member and the second cover member.

12. The applicator for biometric information acquisition of claim 11,
wherein the first cover member is formed from a material having a bacterial barrier property that prevents bacteria from passing through, and breathability, and
wherein the second cover member is formed from a moisture-resistant material.

13. The applicator for biometric information acquisition of claim 11 or 12,
wherein the protection cap includes a first accommodating portion inwardly recessed to accommodate the dehumidifying member and a second accommodating portion accommodating the second cover member and forming a stepped structure with the first accommodating portion.

14. The applicator for biometric information acquisition of any one of the preceding claims,
further comprising an indicator member continuously attached to the protection cap and the case, and
wherein the indicator member is configured to be at least partially broken or to leave a mark on the protection cap or the case when the protection cap is removed.

15. The applicator for biometric information acquisition of claim 14,
wherein the case includes a protruding portion provided to reduce a step difference with one surface of the protection cap by protruding outward and to allow the indicator member to be attached.
